# EUROPÄISCHE PATENTSCHRIFT

(11) **EP 1 646 336 B1**
(45) Veröffentlichungstag und Bekanntmachung des Hinweises auf die Patenterteilung: **08.07.2009**
(21) Anmeldenummer: 03739937.5
(22) Anmeldetag: 22.07.2003
(51) Int. Cl.: A61F 2/44

(54) **ZWISCHENWIRBELIMPLANTAT MIT KALOTTENARTIGEN GELENKFLÄCHEN**
INTERVERTEBRAL IMPLANT COMPRISING DOME-SHAPED JOINT SURFACES
IMPLANT INTERVERTEBRAL COMPORTANT DES SURFACES D'ARTICULATION EN FORME DE CALOTTES

(43) Veröffentlichungstag der Anmeldung: 19.04.2006
(73) Patentinhaber: Synthes GmbH, 4436 Oberdorf (CH)
(72) Erfinder: LECHMANN, Beat, CH-2544 Bettlach (CH); BÜRKI, Roger, CH-4710 Balsthal (CH); FRIGG, Robert, CH-2544 Bettlach (CH)
(74) Vertreter: Lusuardi, Werther
(86) Internationale Anmeldenummer: PCT/CH2003/000495
(87) Internationale Veröffentlichungsnummer: WO 2005/007040

(56) Entgegenhaltungen:
- EP-A- 1 374 807
- WO-A-01/01893
- DE-A- 10 130 798
- US-A- 5 893 889
- US-A1- 2003 074 073
- US-B1- 6 517 580

## Beschreibung

Die Erfindung bezieht sich auf ein Zwischenwirbelimplantat, insbesondere künstliche Bandscheibe, gemäss dem Oberbegriff des Patentanspruchs 1.

Nach Entfernung einer beschädigten, natürlichen Bandscheibe oder eines beschädigten Nukleus pulposus einer Bandscheibe werden heute Implantate oder Prothesen in den Zwischenwirbelraum zweier benachbarter Wirbelkörper eingebracht. Das Ziel der Implantation solcher Implantate ist es, wieder möglichst natürliche Zustände herbeizuführen, d.h. insbesondere die ursprüngliche Bandscheibenhöhe und damit den ursprünglichen Abstand zwischen den beiden benachbarten Wirbelkörpern wiederherzustellen. Ferner sollen Bewegungen der benachbarten Wirbelkörper relativ zueinander möglichst ohne Behinderung in ihrer natürlichen Art ausführbar sein. Hierzu ist die Erhaltung der Bewegungsmöglichkeiten bei einer Vorwärts/Rückwärtsneigung, d.h. Flexion und Extension der Wirbelkörper sowie bei einer lateralen Beugung der Wirbelkörper innerhalb der natürlichen Grenzen wesentlich. Die natürlichen Bänder und Muskeln entlang der Wirbelsäule werden im wesentlichen intakt gelassen, so dass diese die Bewegungen eines mechanischen Bandscheibenersatzes weiter stabilisieren.

Ein solches Zwischenwirbelimplantat ist aus der WO 01/01893 MARNAY bekannt. Dieses bekannte Implantat umfasst eine untere und eine obere Deckplatte, deren Aussenfläche an die Deck- respektive Grundplatte der angrenzenden Wirbelkörper zur Anlage bringbar sind, sowie ein zwischen den Deckplatten angeordnetes Gelenk. Dieses Gelenk besteht im wesentlichen aus einem kalottenförmigen, ersten Gelenkteil und einer komplementären Gelenkschale als zweites Gelenkteil, so dass die Deckplatten relativ zueinander polyaxial schwenkbar sind. Nachteilig an diesem bekannten Zwischenwirbelimplantat ist, dass zwar die zwei Deckplatten je mit einem Gelenkteil verbunden sind, aber die Gelenkteile und somit auch das gesamte Implantat in-situ nur mittels eines speziellen Instrumentes zusammenhaltbar sind.

Aus der US 5,895,428 BERRY ist ein Zwischenwirbelimplantat bekannt, welches Verbindungsmittel zum Zusammenhalten der Gelenkteile umfasst. Nachteilig an diesem bekannten Zwischenwirbelimplantat ist, dass die Verbindungsmittel während einer Relativbewegung der beiden Gelenkteile ebenfalls spielfrei aufeinander gleiten und sich kein Schmierfilm aus körpereigenen Flüssigkeiten aufbauen kann.
Aus der EP 1 374 807 GRlNBERG, die einen Stand der Technik nach Artikel 54(3) darstellt, ist eine Bandscheibenprothese bekannt, welche einteilig mit den Appositionsplatten verbundene Gelenkteile und Mittel zur Begrenzung eines axialen Auseinanderrückens der Gelenkteile umfasst.
Eine weitere künstliche Bandscheibe ist aus der US-A 2003/0074073 ERRICO bekannt. Dieses bekannte umfasst Mittel zum Zusammenhalten des Implantates, welche jedoch kein axiales Spiel zum Aufbau eines Schmierfilmes aus körpereigenen Flüssigkeiten zulassen.

Hier will die Erfindung Abhilfe schaffen. Der Erfindung liegt die Aufgabe zugrunde, ein Zwischenwirbelimplantat zu schaffen, dass ein rotativ bewegbares Gelenk aufweist, dessen Teile vormontierbar sind und mittels Verbindungsmitteln mit Spiel zusammengehalten werden, so dass sich zwischen den Gleitflächen ein Schmierfilm aus körpereigenen Flüssigkeiten aufbauen kann.

Die Erfindung löst die gestellte Aufgabe mit einem Zwischenwirbelimplantat, insbesondere einer künstlichen Bandscheibe, welches die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank des erfindungsgemässen Zwischenwirbelimplantates
- das Gelenk nur ein Gelenkflächenpaar aufweist;
- die miteinander in Eingriff bringbaren Verbindungsmittel bei Schwenkbewegungen der Gelenkteile relativ zueinander im physiologisch erforderlichen Bereich immer Spiel aufweisen und somit die Bewegung der Gelenkteile nicht behindern;
- zwischen den Gleitflächen ein Schmierfilm aus körpereigenen Flüssigkeiten aufgebaut werden kann und damit ein geringer Verschleiss, respektive Abrieb an den Gleitflächen erreichbar ist;
- die Verbindungsmittel, mittels welcher das Zwischenwirbelimplantat zusammengehalten wird, aus geometrisch einfachen Elementen bestehen;
- niedrige Herstellungskosten erreichbar sind; und
- die Gelenkteile mit den Deckplatten verbunden sind, so dass sie sich nicht verschieben und das umliegende Gewebe zerstören können.

In einer bevorzugten Ausführungsform beträgt das Spiel X mindestens 0,005 mm, vorzugsweise mindestens 0,05 mm. Damit ist der Vorteil erreichbar, dass sich ein Schmierfilm der Dicke X aus körpereigenen Flüssigkeiten zwischen den Gleitflächen aufbauen kann. Ferner können die Gelenkflächen mittels zusätzlicher temporärer Fixationsmittel einfacher gegen Beschädigung geschützt werden.

In einer anderen Ausführungsform beträgt das Spiel X maximal 0,5 mm, vorzugsweise maximal 0,6 mm.

In wiederum einer anderen Ausführungsform umfassen die Verbindungsmittel ein mit einem der zwei Teile verbindbares und im anderen der zwei Teile lose gelagertes Verankerungselement. Dadurch sind eine einfache Ausgestaltung der Verbindungsmittel und somit niedrige Herstellungskosten erreichbar.

Vorzugsweise weist das konvexe Gelenkteil einen Hohlraum auf, welcher am virtuellen Apex des konvexen Gelenkteiles offen ist und worin das Verankerungselement axial soweit bewegbar ist, dass es nach Überwindung des Spiels X axial an der Hohlraumwand zur Anlage kommt. Auch hierdurch ist eine einfache Ausgestaltung der Verbindungsmittel und somit niedrige Herstellungskosten erreichbar.

In einer weiteren Ausführungsform umfasst das konvexe Gelenkteil eine sphärische, erste Gelenkfläche A und die Gelenkschale eine dazu komplementäre, zweite Gelenkfläche B. Durch die sphärische Ausgestaltung der Gelenkflächen ist der Vorteil erreichbar, dass die beiden Teile polyaxial relativ zueinander schwenkbar sind.

Die Abmessungen der Gelenkflächen sind je nach Anwendung verschieden, wobei in den verschiedenen Ausführungsformen der Radius der ersten Gelenkfläche A des konvexen sphärischen Gelenkteiles zwischen 3 mm und 25 mm, vorzugsweise zwischen 4 mm und 20 mm beträgt.

In wiederum einer weiteren Ausführungsform bestehen das konvexe Gelenkteil und die Gelenkschale aus einer Metall - Kunststoff Materialpaarung. Damit sind die Vorteile erreichbar, dass
- bereits bewährte Kombinationen von Gelenkersatzmaterialien, wie beispielsweise ein hochvernetztes Polyethylen (X-UHMWPE) und eine Kobalt-Chrom Legierung einsetzbar sind;
- geringe Reibungskräfte bei der relativen Verschiebung der Gleitflächen erreichbar sind; und
- eine Dämpfung für axiale Stossbelastungen erreichbar ist.

In einer anderen Ausführungsform sind die Gelenkflächen A;B mit Titankarbid oder amorphem Kohlenstoff (ADLC) beschichtet, wodurch eine erhebliche Verringerung des Reibungskoeffizienten erreichbar ist.

In wiederum einer anderen Ausführungsform sind die zwei Teile an den Appositionsflächen mit Titan beschichtet.

In einer weiteren Ausführungsform sind die Appositionflächen konvex ausgebildet und an die natürliche Deck- respektive Grundfläche der angrenzenden Wirbelkörper angepasst.

In wiederum einer weiteren Ausführungsform sind die zwei Teile an ihren Appositionsflächen mit makroskopischen Strukturen versehen, welche als Erhebungen ausgebildet sind.

Diese Erhebungen können in verschiedenen Ausführungsformen:
- pyramidenartige Erhebungen umfassen;
- mindestens eine zur Mittelebene symmetrische, keilartige Rippe umfassen, welche sich entlang einer antero-posterioren Geraden auf der betreffenden Appositionsfläche steht; oder
- zur Mittelebene symmetrisch angeordnete, sägezahnartige Zacken umfassen.

Durch diese Ausgestaltungen der makroskopischen Strukturen sind die Vorteile erreichbar, dass einerseits Drehmomente um eine die Appositionsflächen schneidende Drehachse besser von den Wirbelkörpern auf das Zwischenwirbelimplantat übertragen werden können und andererseits die Fläche, woran die Knochen anwachsen können erhöht werden kann. Die Zacken weisen den Vorteil auf, dass sie besser in die Endplatten der angrenzenden Wirbelkörper eindringen können. Die pyramidenförmigen Erhöhungen weisen vorzugsweise ein Volumen V zwischen 0,12 mm³ und 1,4 mm³ auf.

In einer anderen Ausführungsform sind die Erhebungen mindestens teilweise mit Hydroxylapatit oder mit einem bi-phasischen Hydroylapatit-Trikalziumphosphat Gemisch beschichtet, wodurch der Vorteil erreichbar ist, dass beide erwähnte Materialien vollständig in den Knochen integriert oder sogar durch neues, natürliches Knochengewebe ersetzt werden.

Je nach Anwendung sind in verschiedenen Ausführungsformen die untenstehende Geometrie und die folgenden Abmessungen geeignet:
- der Hohlraum ist koaxial zur Zentralachse kreiszylindrisch ausgestaltet;
- der Hohlraum weist einen Durchmesser D zwischen 6 mm und 20 mm aufweist;
- der Hohlraum weist parallel zur Zentralachse gemessen eine Höhe H zwischen 0,5 mm und 8 mm auf;
- der Hohlraum weist eine zur Zentralachse koaxiale Verengung auf, welche in die Gelenkfläche A des konvexen Gelenkteiles mündet;
- die Verengung ist zur Zentralachse koaxial kreiszylindrisch ausgestaltet; und
- die Verengung weist einen Durchmesser d auf, wobei das Verhältnis d : D zwischen 30 % und 75 % beträgt.

Die Erfindung und Weiterbildungen der Erfindung werden im folgenden anhand der teilweise schematischen Darstellungen eines Ausführungsbeispieles noch näher erläutert.

Es zeigen:
Fig. 1 einen Schnitt durch eine Ausführungsform des erfindungsgemässen Zwischenwirbelimplantates;
Fig. 2 eine Ansicht der in Fig. 1 dargestellten Ausführungsform des erfindungsgemässen Zwischenwirbelimplantates;
Fig. 3 eine perspektivische Ansicht der in den Fig. 1 und 2 dargestellten Ausführungsform des erfindungsgemässen Zwischenwirbelimplantates;
Fig. 4 eine Explosionsdarstellung der in den Fig. 1 bis 3 dargestellten Ausführungsform des Zwischenwirbelimplantates;
Fig. 5 eine perspektivische Ansicht des oberen Teiles einer Ausführungsform des Zwischenwirbelimplantates;
Fig. 6 eine Ansicht von lateral auf das obere Teil der in Fig. 5 dargestellten Ausführungsform des Zwischenwirbelimplantates;
Fig. 7 eine Ansicht von lateral auf das obere Teil einer weiteren Ausführungsform des Zwischenwirbelimplantates; und
Fig. 8 eine Ansicht von dorsal auf das obere Teil der in Fig. 7 dargestellten Ausführungsform des Zwischenwirbelimplantates.

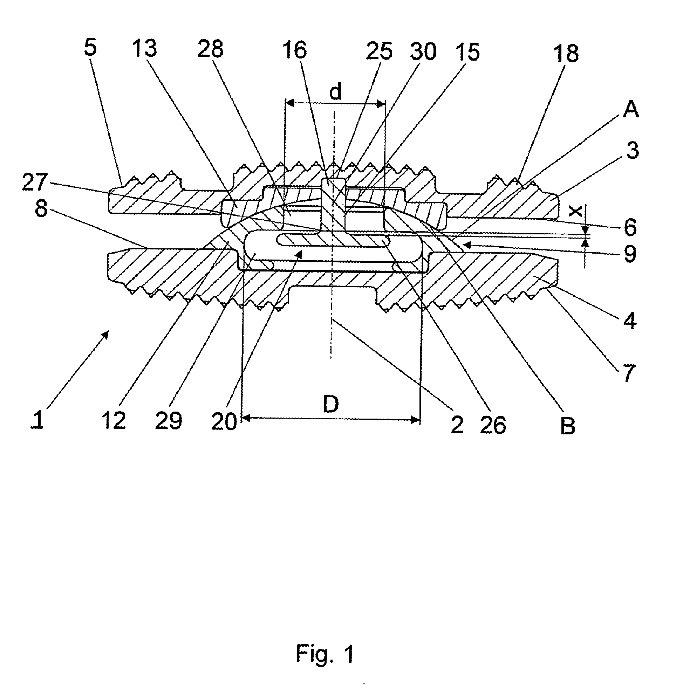

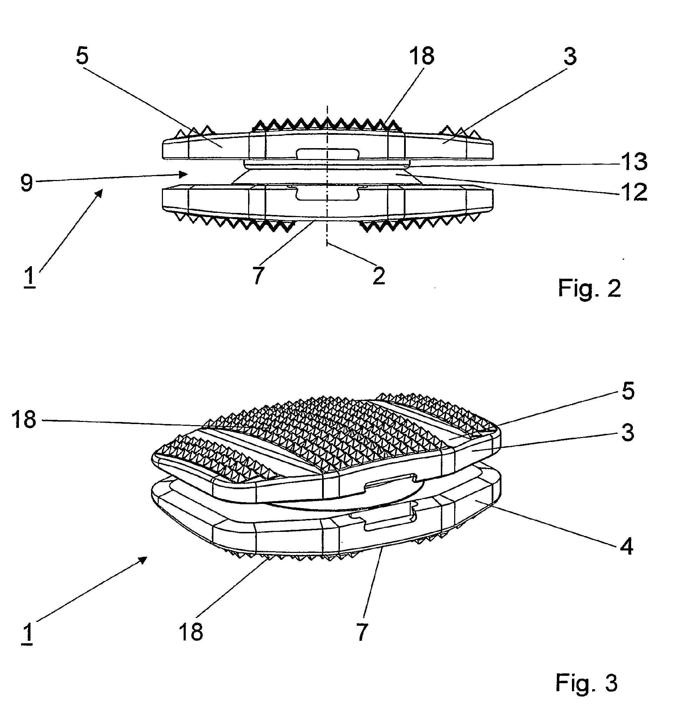

Die in den Fig. 1 bis 4 dargestellte Ausführungsform des erfindungsgemässen Zwischenwirbelimplantates 1 umfasst im wesentlichen ein oberes Teil 3 mit einer die Zentralachse 2 schneidenden, oberen Appositionsfläche 5 zur Anlage an die Grundplatte des oberhalb angrenzenden Wirbelkörpers, ein unteres Teil 4 mit einer die Zentralachse 2 schneidenden, unteren Appositionsfläche 7 zur Anlage an die Deckplatte des unterhalb angrenzenden Wirbelkörpers und ein zwischen den zwei Teilen 3;4 angeordnetes Gelenk 9 zur gelenkigen Verbindung der zwei Teile 3;4. Das Gelenk 9 ist zweiteilig ausgebildet, wobei das konvexe Gelenkteil 12 kugelschichtförmig ausgestaltet ist und mit dem unteren Teil 4 verbunden ist und die zum konvexen Gelenkteil 12 komplementär ausgestaltete Gelenkschale 13 mit dem oberen Teil 3 verbunden ist. Das obere Teil 3 und die Gelenkschale 13 sind in der hier dargestellten Ausführungsform zweiteilig, so dass das obere Teil 3 beispielsweise aus Titan oder einer Kobalt-Chrom Legierung hergestellt und die Gelenkschale 13 beispielsweise aus einem Kunststoff hergestellt sein kann.

Das konvexe Gelenkteil 12 ist zur Zentralachse 2 konzentrisch ausgebildet, wobei es sich gegen die untere Oberfläche 6 des oberen Teiles 3 verjüngt. Die Gelenkschale 13 ist ebenfalls konzentrisch zur Zentralachse 2 angeordnet, wobei die Öffnung der Gelenkschale 13 gegen die obere Oberfläche 8 des unteren Teiles 4 gerichtet ist. Das Verankerungselement 15, wodurch die beiden Teile 3;4 zusammengehalten werden, ist koaxial zur Zentralachse 2 angeordnet und umfasst einen Stift 16, welcher mit seinem hinteren Ende 25 die Gelenkschale 13 beim virtuellen Apex 30 ihrer Oberfläche durchdringt und am oberen Teil 3 befestigt ist, und eine einen grösseren Durchmesser aufweisende kreisscheibenförmige Erweiterung 26 umfasst, welche am vorderen Ende 27 des Stiftes 16 angeordnet ist. Das konvexe Gelenkteil 12 weist eine von seiner verjüngten Seite her eindringende, zur Zentralachse 2 koaxiale Öffnung 28 auf, welche in einen, einen grösseren Durchmesser aufweisenden, zylinderförmigen Hohlraum 29 im Innern des konvexen Gelenkteiles 12 mündet. Die Geometrie des Verankerungselementes 15 sowie der Öffnung 28 und des Hohlraumes 29 ist derart gewählt, dass der Stift 16 in der Öffnung 28 und die Erweiterung 26 im Hohlraum 29 bewegbar angeordnet sind. Der Durchmesser der Öffnung 28 ist kleiner als der Durchmesser der kreisscheibenförmigen Platte 26, so dass das konvexe Gelenkteil 12 und die Gelenkschale 13 und damit die daran befestigten beiden Teile 3;4 axial zusammengehalten werden, ohne dass die Bewegung des Gelenkes 9 behindert wird. Die Bewegbarkeit des Stiftes 16 in der Öffnung 28 und der Platte 26 im Hohlraum 29 ermöglichen eine polyaxiale Schwenkbarkeit der zwei Teile 3;4 relativ zueinander. Die Länge des Stiftes 16 ist so bemessen, dass die Erweiterung 26 am vorderen Ende 27 des Stiftes 16 bei jeder Artikulation des Gelenkes 9 erst nach Überwindung des Spieles X am Übergang zwischen der Öffnung 28 und dem Hohlraum 29 zur Anlage kommt.

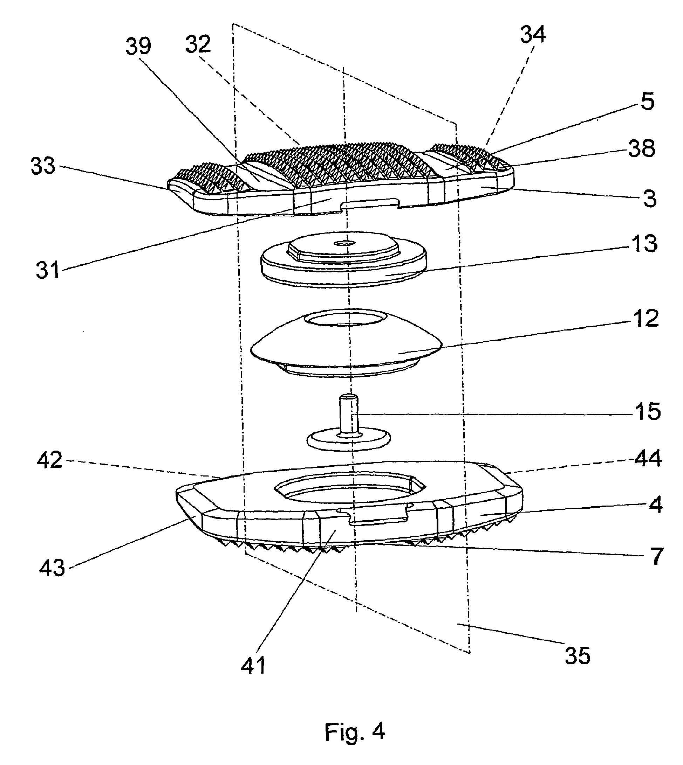

Wie in Fig. 4 dargestellt umfassen die zwei Teile 3;4 je eine ventrale Seitenfläche 31;41, je eine dorsale Seitenfläche 32;42 und je zwei laterale Seitenflächen 33;34;43;44. Ferner sind die zwei Appositionsflächen 5;7 konvex ausgestaltet und mit Erhebungen 18 versehen. Mittig zwischen den lateralen Seitenflächen 33;34;43;44 liegt die Mittelebene 35, welche hier auch die Symmetrieebene für das Zwischenwirbelimplantat 1 bildet. Ferner umfassen die zwei Appositionsflächen 5;7 zwei parallele, zur Mittelebene 35 symmetrisch angeordnete und mindestens in die ventrale Seitenflächen 31;41 mündende Nuten 39, welche beispielsweise zur Aufnahme der Arme eines Distraktionsinstrumentes (nicht gezeichnet) geeignet sind. Die Erhebungen 18 sind hier als pyramidenartige Erhebungen 38 ausgestaltet sind.

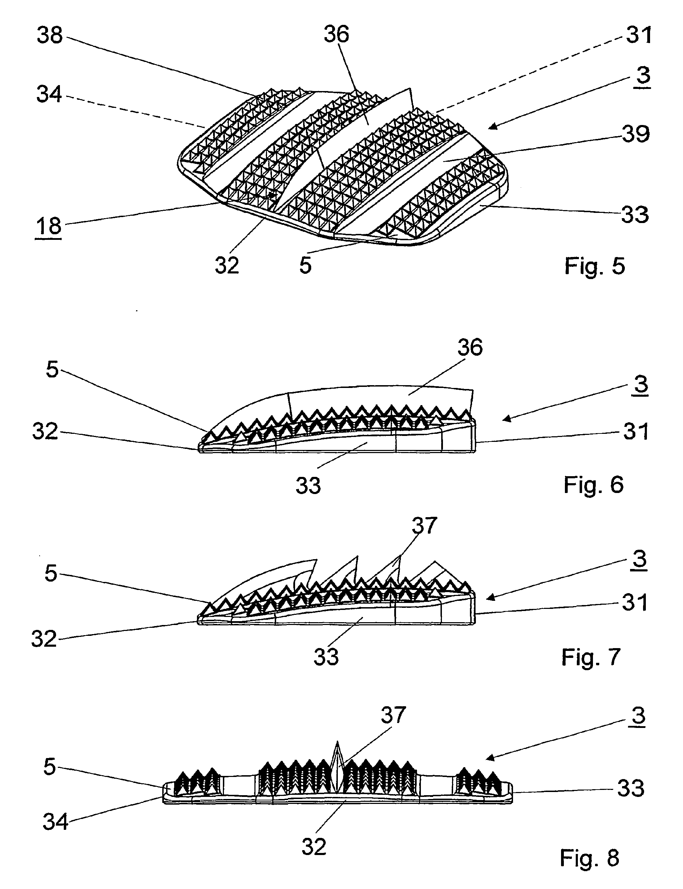

Neben den in Fig. 4 dargestellten pyramidenartigen Erhebungen 38 können die Erhebungen 18 auf der oberen und auf der unteren Appositionsfläche 5;7 wie in den Fig. 5 und 6 dargestellt, je eine keilförmige, gegenüber den pyramidenartigen Erhebungen 38 erhöhte Rippe 36 umfassen, welche parallel zu einer in der Mittelebene 35 (Fig. 4) liegenden Geraden auf den Appositionsflächen 5;7 steht. Damit das Zwischenwirbelimplanat 1 besser in den Zwischenwirbelraum einführbar ist, verringert sich die Höhe der Rippen 36 gegen die dorsale Seitenfläche 32.

Eine weitere Ausführungsform von Erhebungen 18 ist in den Fig. 7 und 8 dargestellt. Die gegenüber den pyramidenförmigen Erhebungen 38 erhöhten Zacken 37 sind teilweise sägezahnförmig ausgestaltet, wobei die steilere Flanke gegen die ventrale Seitenfläche 31 gerichtet ist. Die Zacken 37 liegen hier auf einer in der Mittelebene 35 (Fig. 4) liegenden Geraden und reichen von der ventralen Seitenfläche 31 bis zur dorsalen Seitenfläche 32.

In den Fig. 5 bis 8 ist jeweils nur das obere Teil 3 beispielhaft dargestellt. Dieselbe Ausgestaltung der Erhebungen 18 mit pyramidenförmigen Erhebungen 38, einer Rippe 36 (Fig. 5 und 6) oder Zacken 37 (Fig. 7 und 8) ist analog auf das untere Teil 4 übertragbar.

## Patentansprüche

1. Zwischenwirbelimplantat (1), insbesondere künstliche Bandscheibe, mit einer Zentralachse (2), einem oberen Teil (3) und einem unteren Teil (4), wobei
A) das obere Teil (3) eine obere Appositionsfläche (5) aufweist, welche zur Anlage an die Grundplatte eines darüber liegenden Wirbelkörpers geeignet ist;
B) das untere Teil (4) eine untere Appositionsfläche (6) aufweist, welche zur Anlage an die Deckplatte eines darunter liegenden Wirbelkörpers geeignet ist;
C) eines der zwei Teile (3;4) ein konvexes Gelenkteil (12) mit einer ersten Gelenkfläche A und das andere der zwei Teile (3;4) eine Gelenkschale (13) mit einer auf der ersten Gelenkfläche A gleitbar gelagerten, zweiten Gelenkfläche B umfasst; und
D) das konvexe Gelenkteil (12) und die Gelenkschale (13) ein Gelenk (9) bilden, mittels welchem die zwei Teile (3;4) relativ zueinander um mindestens eine Drehachse rotierbar sind, wobei
E) eines der Teile (3;4) mindestens ein Verbindungsmittel (20) umfasst, wodurch die zwei Teile (3;4) zusammengehalten werden, ohne dass die Schwenkbarkeit des Gelenkes (9) beeinträchtig wird,
**dadurch gekennzeichnet, dass**
F) das Verbindungsmittel (20) bei jeder Artikulation des Gelenkes (9) ein axiales Spiel X > 0 zwischen den zwei Teilen (3;4) und somit auch zwischen den beiden Gleitflächen A und B zulässt;
G) die zwei Teile (3;4) an ihren Appositionsflächen (5;7) mit makroskopischen Strukturen versehen sind; und
H) das obere Teil (3) und die Gelenkschale (13) zweiteilig sind.

2. Zwischenwirbelimplantat (1) nach Anspruch 1, **dadurch gekennzeichnet, dass** das Spiel X mindestens 0,005 mm, vorzugsweise mindestens 0,05 mm beträgt.

3. Zwischenwirbelimplantat (1) nach Anspruch 1 oder 2, **dadurch gekennzeichnet, dass** das Spiel X maximal 0,5 mm, vorzugsweise maximal 0,6 mm beträgt.

4. Zwischenwirbelimplantat (1) nach einem der Ansprüche 1 bis 3, **dadurch gekennzeichnet, dass** die Verbindungsmittel (20) ein mit einem der zwei Teile (3;4) verbindbares und im anderen der zwei Teile (3;4) lose gelagertes Verankerungselement (15) umfassen.

5. Zwischenwirbelimplantat (1) nach Anspruch 4, **dadurch gekennzeichnet, dass** das konvexe Gelenkteil (12) einen Hohlraum (29) aufweist, welcher am virtuellen Apex (30) des konvexen Gelenkteiles (12) offen ist und worin das Verankerungselement (15) axial soweit bewegbar ist, dass es nach Überwindung des Spiels X axial an der Hohlraumwand zur Anlage kommt.

6. Zwischenwirbelimplantat (1) nach einem der Ansprüche 1 bis 5, **dadurch gekennzeichnet, dass** das konvexe Gelenkteil (12) eine sphärische, erste Gelenkfläche A umfasst und die Gelenkschale (13) eine dazu komplementäre, zweite Gelenkfläche B umfasst.

7. Zwischenwirbelimplantat (1) nach Anspruch 6, **dadurch gekennzeichnet, dass** der Radius der ersten Gelenkfläche A des konvexen kalottenartigen Gelenkteiles (12) zwischen 3 mm und 25 mm beträgt.

8. Zwischenwirbelimplantat (1) nach Anspruch 7, **dadurch gekennzeichnet, dass** der Radius der ersten Gelenkfläche A des konvexen kalottenartigen Gelenkteiles (12) zwischen 4 mm und 20 mm beträgt.

9. Zwischenwirbelimplantat (1) nach einem der Ansprüche 1 bis 8, **dadurch gekennzeichnet, dass** das konvexe Gelenkteil (12) und die Gelenkschale (13) aus einer Metall - Kunststoff Materialpaarung bestehen.

10. Zwischenwirbelimplantat (1) nach einem der Ansprüche 1 bis 9, **dadurch gekennzeichnet, dass** die Gelenkflächen A;B mit Titankarbid oder amorphem Kohlenstoff (ADLC) beschichtet sind.

11. Zwischenwirbelimplantat (1) nach einem der Ansprüche 1 bis 10, **dadurch gekennzeichnet, dass** die zwei Teile (3;4) an den Appositionsflächen (5;7) mit Titan beschichtet sind.

12. Zwischenwirbelimplantat (1) nach einem der Ansprüche 1 bis 11, **dadurch gekennzeichnet, dass** die Appositionflächen (5;7) konvex ausgebildet sind.

13. Zwischenwirbelimplantat (1) nach einem der Ansprüche 1 bis 12, **dadurch gekennzeichnet, dass** die makroskopischen Strukturen Erhebungen (18) sind.

14. Zwischenwirbelimplantat (1) nach Anspruch 13, **dadurch gekennzeichnet, dass** die Erhebungen (18) pyramidenartige Erhebungen (38) umfassen

15. Zwischenwirbelimplantat (1) nach Anspruch 13 oder 14, **dadurch gekennzeichnet, dass**
A) die zwei Teile (3;4) je eine ventrale und eine dorsale Seitenfläche (31;41;32;42) sowie je zwei laterale Seitenflächen (33;34;43;44) umfassen;
B) mittig zwischen Seitenflächen (33;34;43;44) eine Mittelebene (35) liegt; und
C) die Erhebungen (1.8) mindestens eine zur Mittelebene (35) symmetrische, keilartige Rippe (36) umfassen, welche parallel zu einer in der Mittelebene (35) liegenden Geraden auf der betreffenden Appositionsfläche 5;7 steht.

16. Zwischenwirbelimplantat (1) nach Anspruch 13 oder 14, **dadurch gekennzeichnet**, das
A) die zwei Teile (3;4) je eine ventrale und eine dorsale Seitenfläche (31;41;32;42) sowie je zwei laterale Seitenflächen (33;34;43;44) umfassen;
B) mittig zwischen Seitenflächen (33;34;43;44) eine Mittelebene (35) liegt; und
C) die Erhebungen (18) zur Mittelebene (35) symmetrisch angeordnete, sägezahnartige Zacken (37) umfassen.

17. Zwischenwirbelimplantat (1) nach einem der Ansprüche 14 bis 16, **dadurch gekennzeichnet, dass** eine pyramidenartige Erhebung (38) ein Volumen V zwischen 0,12 mm³ und 1,4 mm³ aufweist.

18. Zwischenwirbelimplantat (1) nach einem der Ansprüche 13 bis 17, **dadurch gekennzeichnet, dass** die Erhebungen (18) mindestens teilweise mit Hydroxylapatit oder mit einem bi-phasischen Hydroylapatit-Trikalziumphosphat Gemisch beschichtet sind.

19. Zwischenwirbelimplantat (1) nach einem der Ansprüche 5 bis 18, **dadurch gekennzeichnet, dass** der Hohlraum (29) koaxial zur Zentralachse (2) kreiszylindrisch ausgestaltet ist.

20. Zwischenwirbelimplantat (1) nach Anspruch 19, **dadurch gekennzeichnet, dass** der Hohlraum (29) einen Durchmesser D zwischen 6 mm und 20 mm aufweist.

21. Zwischenwirbelimplantat (1) nach Anspruch 19 oder 20, **dadurch gekennzeichnet, dass** der Hohlraum (29) parallel zur Zentralachse (2) gemessen eine Höhe H zwischen 0,5 mm und 8 mm aufweist.

22. Zwischenwirbelimplantat (1) nach einem der Ansprüche 5 bis 21, **dadurch gekennzeichnet, dass** der Hohlraum (29) eine zur Zentralachse (2) koaxiale Öffnung (28) aufweist, welche in die Gelenkfläche A des konvexen Gelenkteiles (12) mündet.

23. Zwischenwirbelimplantat (1) nach Anspruch 22, **dadurch gekennzeichnet, dass** die Öffnung (28) kreiszylindrisch ausgestaltet ist.

24. Zwischenwirbelimplantat (1) nach Anspruch 23, **dadurch gekennzeichnet, dass** die Öffnung (28) einen Durchmesser d aufweist und das Verhältnis d : D zwischen 30 % und 75 % beträgt.

## Claims

1. An intervertebral implant (1) in particular an artificial intervertebral disk, with a central axis (2), a top part (3) and a bottom part (4), wherein
A) the top part (3) has a top apposition surface (5), that is suitable for placing it on the base plate of a body of the vertebra situated above it;
B) the bottom part (4) has a bottom apposition surface (6), that is suitable for placing it on the cover plate of a body of the vertebra situated below it;
C) one of the two parts (3, 4) comprises a convex joint part (12) with a first articular surface A and the other of the two parts (3, 4) comprises a joint shell (13) with a second articular surface B mounted in a sliding manner on the first articular surface A; and
D) the convex joint part (12) and the joint shell (13) form a joint (9), by means of which the two parts (3, 4) can rotate relative one another at least about one axis of rotation, while
E) one of the parts (3, 4) comprises at least one joining means (2), by which the two parts (3, 4) are held together without impairing the capability of the joint (9) to pivot,
**characterised in that**
F) the joining means (20) allows at any articulation of the joint (9) an axial clearance X>0 between the two parts (3, 4) and consequently between the two articular surfaces A and B;
G) on their apposition surfaces (5, 7) the two parts (3, 4) are provided with macroscopic structures; and
H) the top part (3) and the joint shell (13) are made of two parts.

2. An intervertebral implant (1) according to claim 1, **characterised in that** the clearance X is at least 0.005 mm, preferably at least 0.05 mm.

3. An intervertebral implant (1) according to claim 1 or 2, **characterised in that** the clearance X is maximum 0.5 mm, preferably maximum 0.6 mm.

4. An intervertebral implant (1) according to any one of claims 1 to 3, **characterised in that** the joining means (20) comprise an anchoring means (15) that can be joined with one of the two parts (3, 4) and is loosely mounted in the other of the two parts (3, 4).

5. An intervertebral implant (1) according to claim 4, **characterised in that** the convex joint part (12) has a hollow space (29), that is open on the virtual apex (30) of the convex joint part (12) and in which the anchoring element (15) can be axially displaced until it comes to a rest on the wall of the hollow space after overcoming the axial clearance X.

6. An intervertebral implant (1) according to any one of claims 1 to 5, **characterised in that** the convex joint part (12) has a spherical first articular surface A and the joint shell (13) a second articular surface B, matching the first one.

7. An intervertebral implant (1) according to claim 6, **characterised in that** the radius of the first articular surface A of the convex hemispherical joint part (12) is between 3 mm and 25 mm.

8. An intervertebral implant (1) according to claim 7, **characterised in that** the radius of the first articular surface A of the convex hemispherical joint part (12) is between 4 mm and 20 mm.

9. An intervertebral implant (1) according to any one of claims 1 to 8, **characterised in that** the convex joint part (12) and the joint shell (13) are made from a metal/plastics material pair.

10. An intervertebral implant (1) according to any one of claims 1 to 9, **characterised in that** the articular surfaces A, B are coated with titanium carbide or amorphous carbon (ADLC).

11. An intervertebral implant (1) according to any one of claims 1 to 10, **characterised in that** the two parts (3, 4) are coated with titanium on the apposed surfaces (5, 7).

12. An intervertebral implant (1) according to any one of claims 1 to 11, **characterised in that** the apposition surfaces (5, 7) have a convex construction.

13. An intervertebral implant (1) according to any of claims 1 to 12, **characterised in that** the macroscopic structures are protuberances (18).

14. An intervertebral implant (1) according to claim 13, **characterised in that** the protuberances (18) comprise pyramid-like protuberances (38).

15. An intervertebral implant (1) according to claim 13 or 14, **characterised in that**
A) the two parts (3, 4) comprise a ventral and a dorsal lateral surface (31, 41, 32, 42) as well as two lateral surfaces (33, 34, 43, 44), respectively;
B) between the lateral side surfaces (33, 34, 43, 44) in the middle there is a central plane (35); and
C) the protuberances (18) comprise at least one wedge-shaped rib (36) that is symmetrical about the central plane (35) and is situated on a straight line situated in the central plane (35) on the respective apposition surfaces (5, 7).

16. An intervertebral implant (1) according to claim 13 or 14, **characterised in that**
A) the two parts (3, 4) comprise a ventral and a dorsal lateral surface (31, 41, 32, 42) as well as two lateral surfaces (33, 34, 43, 44), respectively;
B) between the lateral side surfaces (33, 34, 43, 44) in the middle there is a central plane (35); and
C) the protuberances (18) comprise saw-tooth like serrations (37) arranged symmetrically about the central plane (35).

17. An intervertebral implant (1) according to any one of claims 14 to 16, **characterised in that** a pyramid-like protuberance (38) has a volume V between 0.12 mm³ and 1.4 mm³.

18. An intervertebral implant (1) according to any one of claims 13 to 17, **characterised in that** the protuberances (18) are coated at least partly with hydroxylapatite or with a bi-phased hydroxylapatite-tricalcium phosphate mixture.

19. An intervertebral implant (1) according to any one of claims 4 to 18, **characterised in that** the hollow space (29) is circular cylindrical and coaxial with the central axis (2).

20. An intervertebral implant (1) according to claim 19, **characterised in that** the hollow space (29) has a diameter D between 6 mm and 20 mm.

21. An intervertebral implant (1) according to claim 19 or 20, **characterised in that** when measured parallel to the central axis (2) the hollow space (29) has a height H between 0.5 mm and 8 mm.

22. An intervertebral implant (1) according to any one of claims 4 to 21, **characterised in that** the hollow space (29) has a opening (28) that is coaxial with the central axis (2) and terminates in the articular surface A of the convex joint part (12).

23. An intervertebral implant (1) according to claim 22, **characterised in that** the opening (28) is circular cylindrical.

24. An intervertebral implant (1) according to claim 23, **characterised in that** the opening (28) has a diameter d and the ratio of d:D is between 30% and 75%.

## Revendications

1. Implant vertébral (1), notamment disque vertébral artificiel, comportant un axe central (2), une pièce supérieure (3) et une pièce inférieure (4),
A) la pièce supérieure (3) présentant une surface d'apposition supérieure (5) qui est appropriée pour porter sur la surface articulaire inférieure d'un corps vertébral sus-jacent ;
B) la pièce inférieure (4) présentant une surface d'apposition inférieure (6) qui est appropriée pour porter sur la surface articulaire supérieure d'un corps vertébral sous-jacent ;
C) l'une des deux pièce (3 ; 4) comportant une pièce d'articulation convexe (12) présentant une première surface d'articulation A et l'autre des deux parties (3 ; 4) comportant une coque d'articulation (13) présentant une deuxième surface d'articulation B montée de façon à pouvoir glisser sur la première surface d'articulation A ; et
D) la pièce d'articulation convexe (12) et la coque d'articulation (13) formant une articulation (9) qui permet aux deux pièces (3 ; 4) de tourner l'une par rapport à l'autre autour d'au moins un axe de rotation,
E) l'une des pièces (3 ; 4) comportant au moins un moyen de liaison (20) qui permet de maintenir ensemble les deux pièces (3 ; 4) sans nuire à la capacité de pivotement de l'articulation (9),
**caractérisé en ce que**
F) le moyen de liaison (20) autorise, à chaque mouvement articulé de l'articulation (9), un jeu axial X > 0 entre les deux pièces (3 ; 4) et donc également entre les deux surfaces de glissement A et B ;
G) les deux pièces (3 ; 4) sont dotées de structures macroscopiques au niveau de leur surface d'apposition (5 ; 7) ; et
H) la pièce supérieure (3) et la coque d'articulation (13) sont en deux pièces.

2. Implant vertébral (1) selon la revendication 1, **caractérisé en ce que** le jeu X est d'au moins 0,005 mm, avantageusement d'au moins 0,05 mm.

3. Implant vertébral (1) selon la revendication 1 ou 2, **caractérisé en ce que** le jeu X est de 0,5 mm maximum, avantageusement de 0,6 mm maximum.

4. Implant vertébral (1) selon l'une des revendications 1 à 3, **caractérisé en ce que** les moyens de liaison (20) comportent un élément d'ancrage (15) qui peut être relié à l'une des deux pièces (3 ; 4) et qui est logé sans serrage dans l'autre des deux pièces (3 ; 4).

5. Implant vertébral (1) selon la revendication 4, **caractérisé en ce que** la pièce d'articulation convexe (12) comporte une cavité (29) qui est ouverte au niveau du sommet virtuel (30) de la pièce d'articulation convexe (12) et dans laquelle l'élément d'ancrage (15) peut se déplacer axialement jusqu'à venir axialement en appui contre la paroi de la cavité au-delà du jeu X.

6. Implant vertébral (1) selon l'une des revendications 1 à 5, **caractérisé en ce que** la pièce d'articulation convexe (12) présente une première surface d'articulation sphérique A et la coque d'articulation (13) présente une deuxième surface d'articulation B complémentaire.

7. Implant vertébral (1) selon la revendication 6, **caractérisé en ce que** le rayon de la première surface d'articulation A de la pièce d'articulation convexe (12) en forme de calotte est compris entre 3 mm et 25 mm.

8. Implant vertébral (1) selon la revendication 7, **caractérisé en ce que** le rayon de la première surface d'articulation A de la pièce d'articulation convexe (12) en forme de calotte est compris entre 4 mm et 20 mm.

9. Implant vertébral (1) selon l'une des revendications 1 à 8, **caractérisé en ce que** la pièce d'articulation convexe (12) et la coque d'articulation (13) sont en matériaux appariés métal/matière plastique.

10. Implant vertébral (1) selon l'une des revendications 1 à 9, **caractérisé en ce que** les surfaces d'articulation A ; B sont dotées d'un revêtement en carbure de titane ou en carbone amorphe (ADLC).

11. Implant vertébral (1) selon l'une des revendications 1 à 10, **caractérisé en ce que** les deux pièces (3 ; 4) sont dotées d'un revêtement en titane au niveau des surfaces d'apposition (5 ; 7).

12. Implant vertébral (1) selon l'une des revendications 1 à 11, **caractérisé en ce que** les surfaces d'apposition (5 ; 7) ont une conformation convexe.

13. Implant vertébral (1) selon l'une des revendications 1 à 12, **caractérisé en ce que** les structures macroscopiques sont des saillies (18).

14. Implant vertébral (1) selon la revendication 13, **caractérisé en ce que** les saillies (18) incluent des saillies pyramidales (38).

15. Implant vertébral (1) selon la revendication 13 ou 14, **caractérisé en ce que**
A) les deux pièces (3 ; 4) présentent chacune des faces ventrale et dorsale (31 ; 41 ; 32 ; 42) ainsi que deux faces latérales (33 ; 34 ; 43 ; 44) ;
B) un plan médian (35) passe au milieu entre les faces (33 ; 34 ; 43 ; 44) ; et
C) les saillies (18) comportent au moins une nervure cunéiforme (36) qui est symétrique par rapport au plan médian (35) et qui est placée sur ladite surface d'apposition (5 ; 7) parallèlement à une droite du plan médian (35).

16. Implant vertébral (1) selon la revendication 13 ou 14, **caractérisé en ce que**
A) les deux pièces (3 ; 4) présentent chacune des faces ventrale et dorsale (31 ; 41 ; 32 ; 42) ainsi que deux faces latérales (33 ; 34 ; 43 ; 44) ;
B) un plan médian (35) passe au milieu entre les faces latérales (33 ; 34 ; 43 ; 44); et
C) les saillies (18) comportent des pointes (37), en forme de dents de scie, qui sont disposées symétriquement par rapport au plan médian (35).

17. Implant vertébral (1) selon l'une des revendications 14 à 16, **caractérisé en ce qu'**une saillie pyramidale (38) a un volume V compris entre 0,12 mm³ et 1,4 mm³.

18. Implant vertébral (1) selon l'une des revendications 13 à 17, **caractérisé en ce que** les saillies (18) sont revêtues au moins partiellement d'hydroxylapatite ou d'un mélange biphasique d'hydroxylapatite et de phosphate tricalcique.

19. Implant vertébral (1) selon l'une des revendications 5 à 18, **caractérisé en ce que** la cavité (29) est conformée, coaxialement à l'axe central (2), en cylindre circulaire.

20. Implant vertébral (1) selon la revendication 19, **caractérisé en ce que** la cavité (29) a un diamètre D compris entre 6 mm et 20 mm.

21. Implant vertébral (1) selon la revendication 19 ou 20, **caractérisé en ce que** la cavité (29) a une hauteur H, mesurée parallèlement à l'axe central (2), qui est comprise entre 0,5 mm et 8 mm.

22. Implant vertébral (1) selon l'une des revendications 5 à 21, **caractérisé en ce que** la cavité (29) comporte une ouverture (28) qui est coaxiale à l'axe central (2) et qui débouche dans la surface d'articulation A de la pièce d'articulation convexe (12).

23. Implant vertébral (1) selon la revendication 22, **caractérisé en ce que** l'ouverture (28) est conformée en cylindre circulaire.

24. Implant vertébral (1) selon la revendication 23, **caractérisé en ce que** l'ouverture (28) a un diamètre d et le rapport d : D est compris entre 30% et 75%.
